# EUROPÄISCHE PATENTSCHRIFT

(11) **EP 2 405 924 B1**
(45) Veröffentlichungstag und Bekanntmachung des Hinweises auf die Patenterteilung: **19.12.2012**
(21) Anmeldenummer: 10707043.5
(22) Anmeldetag: 08.03.2010
(51) Int. Cl.: A61K 33/00, A61K 33/14, A61K 33/42, A61L 2/00, A61K 9/00, A61P 3/12, A61P 3/00, A61P 43/00

(54) **PLASMAADAPTIERTE VOLLELEKTROLYTLÖSUNG**
PLASMA-ADAPTED FULL ELECTROLYTE SOLUTION
SOLUTION ÉLECTROLYTIQUE COMPLÈTE ADAPTÉE AU PLASMA

(30) Priorität: 13.03.2009 DE 102009012671
(43) Veröffentlichungstag der Anmeldung: 18.01.2012
(73) Patentinhaber: B. Braun Melsungen AG, 34212 Melsungen (DE)
(72) Erfinder: BOLL, Michael, 34212 Melsungen (DE)
(74) Vertreter: von Kreisler Selting Werner
(86) Internationale Anmeldenummer: PCT/EP2010/052895
(87) Internationale Veröffentlichungsnummer: WO 2010/102972

(56) Entgegenhaltungen:
- EP-A1- 0 613 688
- WO-A1-01/94349
- US-A- 4 308 255
- US-A- 5 955 257
- Boldt J: "Saline versus balanced hydroxyethyl starch: does it matter?" Transfusion Alternatives in Transfusion Medicine Bd. 9, Nr. 3, September 2007 (2007-09), Seiten 189-197, XP002584617 ISSN: 1295-9022 DOI: 10.1111/j.1778-428X.2007.00073.x Gefunden im Internet: URL:http://www3.interscience.wiley.com/cgi -bin/fulltext/118489782/PDFSTART> [gefunden am 2010-10-27] in der Anmeldung erwähnt
- ANDREU G ET AL: "Introduction of platelet additive solutions in transfusion practice. Advantages, disadvantages and benefit for patients" TRANSFUSION CLINIQUE ET BIOLOGIQUE - XXIIIE CONGRES NATIONAL DE LA SFTS, 2-5 JUILLET 2007 - TOURS 200705 FR LNKD- DOI:10.1016/J.TRACLI.2007.03.009, Bd. 14, Nr. 1, Mai 2007 (2007-05), Seiten 100-106, XP002584618 ISSN: 1246-7820
- DATABASE WPI Week 199429 Thomson Scientific, London, GB; AN 1994-238655 XP002584619 & JP 06 172191 A (GREEN CROSS CORP) 21. Juni 1994 (1994-06-21)

## Beschreibung

Die vorliegende Erfindung bezieht sich auf eine wässrige Vollelektrolytlösung. Die vorliegende Erfindung bezieht sich insbesondere auf eine wässrige Vollelektrolytlösung, die sich aufgrund ihrer Plasmaadaptierung besonders ais Lösung zur intravenösen und zur subkutanen Infusion eignet. Dabei wird neben der Vollelektrolytlösung selber auch ein Elektrolytgemisch nebst einem Verfahren der Herstellung der Vollelektrolytlösung daraus sowie ein Arzneimittel, das die Lösung sowie gegebenenfalls einen Wirkstoff umfasst, bereit gestellt.

Die intravenöse Verabreichungen von Salzlösungen an Patienten ("Tropf") ist die häufigste Infusionsbehandlung, die im präklinischen Bereich von Notfällen und Unfällen, im klinischen Bereich (dort überall), in Praxen von niedergelassenen Ärzten sowie zunehmend in Senioren-Pflegeeinrichtungen durchgeführt wird. Dieses geschieht dann, wenn bei den Patienten ein akuter Flüssigkeitsmangel des extrazellulären Raums besteht, den die Patienten nicht schnell genug durch Trinken beheben können, wollen oder sollen, wie z.B. aufgrund einer Störung des Durstempfindens oder einer erforderlichen Ruhigstellung des Magen-DarmTraktes. Insbesondere werden solche Salzlösungen infundiert, um akute Flüssigkeitsverluste, die z.B. durch Erbrechen oder Diarrhöen hervorgerufen wurden, oder schockbedingte Flüssigkeitsdefizite im Kreislauf bzw. Blutverluste auszugleichen. Letzteres geschieht zum Teil nur vorübergehend, bis kolloidale Volumenersatzlösungen oder Blutpräparate zur Verfügung stehen. Bei den in allen diesen Fällen zu verwendenden Salzlösungen handelt es sich vorzugsweise um Vollelektrolytlösungen, d. h. um Lösungen, die im Idealfall sämtliche Elektrolyte enthalten sollten, wie sie auch im menschlichen Blutplasma vorkommen, und zwar in den dort vorliegenden Mengenverhältnissen sowie osmotisch wirksamen Konzentrationen.

Des weiteren werden solche Salzlösungen in großem Umfang dafür genutzt, um Medikamente, Nähr- oder zusätzliche Mineralstoffe, die intravenös verabreicht werden sollen, darin aufzulösen oder zu verdünnen und sie mit definierter gleichbleibender Geschwindigkeit zu infundieren ("Trägerlösungen").

Solche Infusionstherapien im heutigen Sinne sind erst im 20. Jahrhundert realisiert worden und auch seit dem 2. Weltkrieg in breitem Rahmen üblich geworden. Dabei wurden allerdings lange Zeit die Infusionslösungen in Europa von den Kliniken vor der Verwendung selber gemischt und zur Sterilisierung erhitzt. Später wurden die Lösungen standardisiert und von den jeweiligen Klinikapotheken zubereitet. In den letzten drei Jahrzehnten ist dieses hauptsächlich aus Kostengründen durch die Industrie vorgenommen worden (W. Druml, Wien. Klin Wochenschr. 2005, 117/3, 67-70*).*

Die Herausforderung bei der Herstellung und Formulierung solcher Vollelektrolytlösungen zur Substitution von extrazellulärer Flüssigkeit und Elektrolyten besteht darin, dass einerseits das physiologische Elektrolytmuster des Plasmas weitgehend nachzubilden ist, und zwar - entsprechend ihrer Bedeutung - die Konzentration der Kationen (Natrium, Kalium, Calcium und Magnesium) und der Anionen (Chlorid, Phosphat und Bicarbonat) [Zander et al., Anästhesiol. Intensivmed. Notfallmed. Schmerzther. 2005, 40, 701-719], andererseits aber Einschränkungen zu berücksichtigen sind, die sich von technologischer und klinischer Seite her ergeben. Aus galenischen und anderen Gründen wie insbesondere der Instabilität von Bicarbonat bei der Hitzesterilisation und Verwendung in gewöhnlichen Kunststoffbehältern lässt sich bereits die Forderung nach einem plasmaidentischen Bicarbonatgehalt nur bedingt erfüllen. Letztlich gelingt dies dadurch, dass anstelle von Bicarbonat bzw. dessen theoretisch geeigneten Salzen als solchen Bicarbonatvorstufen eingesetzt werden. Zu diesem Zweck eignen sich organische Anionen wie Laktat, Acetat oder andere, die erst bei der Infusion im Organismus zu Bicarbonat verstoffwechselt werden und auf diese Weise bereitstellen. Abgesehen von der mit dem Fehlen bzw. dem Ersatz von Bicarbonat verbundenen Problematik sollte die Osmolalität der betreffenden Lösungen mit derjenigen des Blutplasmas übereinstimmen, damit unerwünschte Flüssigkeitsverschiebungen zwischen intra - und extrazellulären Räumen vermieden werden. Außerdem dürfen die zur intravenösen Infusion bestimmten Lösungen zwecks Gewährleistung guter lokaler Verträglichkeit bei der Infusion keine nennenswerte Azidität oder Alkalität besitzen, und sie müssen darüberhinaus so zusammengesetzt sein, dass sie den physiologischen Säure-Basen-Haushalt nicht verändern. Und schließlich besteht das Problem, dass das Blutplasma bestimmte Eiweißkörper enthält, die selbst keine Bestandteile von Vollelektrolytlösungen darstellen, deren negative Ladungen aus Gründen der Elektroneutralität aber die Anwesenheit von Gegenionen erfordert, die in den eiweißfreien Elektrolytlösungen nicht in gleicher Weise nachzubilden ist.

Alle derzeit in Gebrauch befindlichen Vollelektrolytlösungen stellen daher einen Kompromiss zwischen der Forderung nach einem idealen plasmaidentischen und dem in der Lösung real vorhandenen Elektrolytmuster dar und erfüllen die übrigen genannten Anforderungen nicht oder nur unvollständig. Die Diskrepanz zwischen idealer und realer Zusammensetzung wird dadurch begünstigt, dass jede verstärkte Bemühung auf die Erfüllung einzelner Elemente zwangsläufig zur Vernachlässigung anderer Elemente führen muß.

Bedingt durch die oben angesprochene historische Entwicklung, in deren Verlauf auf lokaler Ebene Infusionslösungen im Klinikbereich selber hergestellt wurden, existiert nun eine Vielzahl von Infusionslösungen unterschiedlicher Zusammensetzung, die - jede auf ihre Weise - den genannten Problemen gerecht zu werden versuchen. Dabei hat sich zunächst auch schon aus produktionstechnischen Gründen wie einfacher Herstellung und niedrigen Kosten eine 0,9 % Natriumchlorid-Lösung als die am häufigsten angewendete Infusionslösung in der Infusionstherapie durchgesetzt. Des weiteren existieren Lösungen, die neben Natriumchlorid noch weitere Elektrolyte enthalten, wie die nach ihren Schöpfern benannte Ringer- und Hartmann-Lösungen sowie die Ringer-Laktat-Lösung (RL), die wie die Hartmann-Lösung Laktat-Ionen enthält. In Tabelle 1 sind die Zusammensetzungen dieser Infusionslösungen dem Elektrolytgehalt des humanen Blutplasmas gegenüber gestellt (J. Boldt, Transfusion Alternatives in Transfusion Medicine, 2007, 9, 189-197).

**Tabelle 1: Zusammensetzungen verschiedener Infusionslösungen. Die einzelnen Zusammensetzungen variieren dabei nach Hersteller und Ursprungsland.**

| **Elektrolyte** | **Plasma** | **0,9 % NaCl** | **Ringer** | **RL** | **PlasmaLyte 148** | **Hartmann** |
|---|---|---|---|---|---|---|
| Na⁺ [mmol/l] | 140 | 154 | 154 | 131 | 140 | 129 |
| K⁺ [mmol/l] | 4,2 | - | 4,0 | 5,4 | 5 | 5 |
| Ca²⁺ [mmo/l] | 2,5 | - | 2,3 | 1,8 | - | 4 |
| Mg²⁺ [mmol/l] | 3 | - | - | 0,5 | 3 | - |
| Phosphat [mmol/ll | 1,25 | - | - | - | - | - |
| Cl⁻ [mmol/l] | 103 | 154 | 163 | 112 | 98 | 109 |
| Lactat [mmol/l] | 1 | - | - | 27 | - | 29 |
| Acetat [mmol/l] | - | - | - | - | 27 | - |
| Na⁺/Cl⁻-Verhältnis | 1,36 | 1,0 | 0,94 | 1,17 | 1,42 | 1,18 |

Wie aus Tabelle 1 im Vergleich mit der Zusammensetzung des Plasmas hervorgeht, sind die angeführten Lösungen zum Teil nicht "physiologisch", da sie aus einer nicht-physiologischen, d. h. den Mengenverhältnissen im Blutplasma nicht entsprechenden, Mischung von Elektrolyten bestehen oder eine vom Blutplasma verschiedene Osmolalität oder sonstige Unterschiede zeigen.

Darüber hinaus sind noch teilweise erhebliche Nebenwirkungen dieser Lösungen bekannt, beziehungsweise wurde über Komplikationen beim Einsatz der Infusionslösungen berichtet. So werden beträchtliche Veränderungen im Säure-Base-Haushalt von Patienten beobachtet, denen große Mengen an solchen Salzlösungen infundiert werden, die kein Bicarbonat enthalten bzw. bei denen der Chlorid- im Vergleich zum Natriumanteil über dem des Blutplasmas liegt. Dieses Phänomen wird - je nach Betrachtungsweise - als "Dilutionsazidose" oder als "hyperchlorämische Azidose" klassifiziert. Wie bereits oben gesagt lässt sich das Fehlen von Bicarbonat zwar durch den Einsatz von bestimmten organischen Anionen kompensieren, die zu Bicarbonat verstoffwechselt werden und es auf sekundäre Weise substituieren, jedoch sind die dazu erforderlichen Mengen von organischen Anionen geringer als es wünschenswert wäre, um den Anteil von anorganischen Anionen wie Chlorid auf ein plasmaidentisches Maß senken zu können. Daher lässt sich eine hyperchlorämische Azidose bei partiellem Austausch von Chlorid-Anionen durch bicarbonatliefernde organische Anionen entweder nicht ganz verhindern, oder es besteht - bei Verwendung höherer Konzentrationen von bicarbonatliefernden Anionen - die Gefahr, dass eine Alkalose als Folge der Infusion inadäquat zusammengesetzter Infusionslösungen eintritt.

Andere Komplikationen haben ihre Ursache z. T. darin, dass die Osmolalität vieler derzeit gebräuchlicher Elektrolytlösungen unter der des Blutplasmas liegt und damit vermehrt sogenanntes "freies Wasser" zugeführt wird. Unter "freiem Wasser" versteht man Flüssigkeit, die nicht durch einen entsprechenden Ionenanteil gebunden ist. Bei der intravenösen Infusion dringt "freies Wasser" vermehrt in Körpergewebe ein und begünstigt dort die Bildung von Flüssigkeitsansammlungen ("Ödemen"). Hirnzellen reagieren besonders empfindlich auf Osmolalitätsänderungen, was sich in zerebralen Symptomen äußert, die von Somnolenz bis hin zur Enzephalopathie oder zum Koma reichen. Speziell bei Früh- und Neugeborenen, bei denen der Anteil der Hirnmasse am Körpergewicht im Unterschied zum Erwachsenen überproportional hoch ist, kann es besonders leicht zum Hirnödem kommen. Bei Zufuhr grösserer Mengen von "freiem Wasser" in Form hypotoner Infusionslösungen bzw. solcher mit zu niedriger Osmolalität im Vergleich mit der des Blutplasmas wurden gerade in der Pädiatrie sogar Todesfälle beschrieben (A. I. Arieff, Paediatric Anaesthesia, 1998, 8, 1-4). Besonders gefährdet sind auch Patienten mit einem Schädel-Hirn-Trauma, weil bei ihnen das Risiko der Verstärkung eines Hirnödems bzw. eines Hirndruckanstiegs besteht, wenn sie mit hypotonen Infusionslösungen behandelt werden. Typischer Vertreter einer hypotonen Infusionslösung ist z. B. die weit verbreitete Ringer-Laktat-Lösung. Ein weiterer mit möglichen Komplikationen verbundener Nachteil dieses Lösungstyps besteht darin, dass das in der Lösung enthaltene Laktat nicht zu Bicarbonat verstoffwechselt werden kann, wenn eine schwere Leberschädigung vorliegt, wie es bei schwerkranken Intensivpatienten oder solchen im schweren Schock nicht selten der Fall ist.

Des Weiteren sind unerwünschte Nebenwirkungen beim Infundieren kommerziell erhältlicher Infusionslösungen wie schlechte lokale Verträglichkeiten sowie Probleme der Kompatibilität mit zugemischten Arzneimitteln bekannt. Die Entwicklung und Herstellung von zur Infusion geeigneter Elektrolytlösungen, die dem physiologischen Elektrolytprofil des Plasmas entsprechen, sich im Herstellungsverfahren hitzesterilisieren lassen, eine gute lokale Verträglichkeit und hohe Kompatibilität mit zugesetzten Medikamenten aufweisen, die gleiche Osmolalität wie das Plasma besitzen und den Säure-Basen-Haushalt unbeeinflusst lassen, ist somit Gegenstand der universitären und kommerziellen Forschung.

So beschreibt DE 32 24 823 A1 ein Verfahren zur Herstellung einer für den entsprechenden Krankheitsfall optimierten Elektrolytlösung zur Anwendung in der Hämodialyse. Dabei wird von 9 I einer Stammlösung ausgegangen, die 45,5 val Natrium (Na⁺), 350 mval Magnesium (Mg⁺⁺), 12,25 val Acetat (CH₃CO₂⁻) und 33,6 val Chlorid (Cl⁻) sowie gegebenenfalls 630 bis 720 g Glucose enthält. Der gewünschte Endgehalt wird dadurch eingestellt, dass man das auf 10 l fehlende Volumen mit den gewünschten Konzentrationsabstufungen entsprechenden sterilen Einheiten an 3,5 N Natriumchlorid-, Kaliumchlorid-, Calciumchlorid und/oder Magnesiumacetatlösungen auffüllt.

EP 0 613 688 B2 offenbart ein Verfahren, das die individuelle Adaptierung der Zusammensetzung der Dialyseflüssigkeit an unterschiedliche therapeutische Anforderungen durch die Verwendung eines Grundkonzentrats, welches hauptsächlich Natriumchlorid und Natriumhydrogencarbonat umfasst, ermöglicht.

Aus den oben genannten Gründen war die Schaffung einer plasmaadaptierten, isotonen und balancierten Vollelektrolytlösung, welche die Nachteile bereits bestehender Vollelektrolytlösungen so weit als möglich vermeidet, wünschenswert. Aufgabe der vorliegenden Erfindung war es daher, eine isotone, plasmaadaptierte Vollelektrolytlösung bereit zu stellen, die nicht zu infusionsbedingten Störungen der Homöostase des Elektrolyt- und Säure-Basen-Haushaltes führt und eine gute lokale Verträglichkeit aufweist. Überraschend wurde gefunden, dass die Aufgabe durch eine wässrige Vollelektrolytlösung, wie sie in Anspruch 1 definiert ist, gelöst wird. Es wurde zudem überraschend gefunden, dass die erfindungsgemäße Elektrolytlösung die zuvor genannten Probleme des Standes der Technik löst und insbesondere keine lokale Reizungen der Blutgefäße bzw. des infusionsnahen Gewebes induziert.

Ein erster Gegenstand der vorliegenden Erfindung ist demgemäss eine wässrige Vollelektrolytlösung, umfassend die Ionenanteile a) 138 - 146 mmol/l Natrium, b) 4 -5 mmol/l Kalium, c) 0.5 - 2.0 mmol/l Calcium, d) 1.0 - 1.5 mmol/l Magnesium, e) 100 -108 mmol/l Chlorid, f) 0.5 - 1.5 mmol/l Phosphat, g) 18 - 26 mmol/l Glukonat und h) 20 -28 mmol/l Acetat.

Bevorzugte Ausführungsformen der erfindungsgemäßen Vollelektrolytlösung sind dabei die Ionenanteile, die jeweils unabhängig voneinander in wenigstens einer der folgenden Konzentrationen vorliegen: a) 140 - 144 mmol/l Natrium und/oder b) 4,3 - 4,7 mmol/l Kalium und/oder c) 1,0 - 1,5 mmol/l Calcium und/oder d) 1,1 - 1,4 mmol/l Magnesium und/oder e) 102 - 106 mmol/l Chlorid und/oder f) 0,8 - 1,2 mmol/l Phosphat und/oder g) 20 - 24 mmol/l Glukonat und/oder h) 22 - 26 mmol/l Acetat.

Bevorzugt ist weiterhin, dass die wässrige Vollelektrolytlösung einen pH-Wert im Bereich von 5,0 bis 8,0, besonders bevorzugt von 6,0 bis 7,0, aufweist. Weiter bevorzugt weist die erfindungsgemäße Vollelektrolytlösung eine Osmolalität von 280 - 300 mosmol/kg H₂O auf.

Es hat sich als vorteilhaft herausgestellt, Elektrolytmischungen in fester Form zusammenzustellen, die durch Lösen in Wasser zu erfindungsgemäßen Elektrolytlösungen führen können. Dieses vereinfacht den Transport, da das Gewicht und Volumen des Lösungsmittels nicht mit transportiert werden müssen. Des weiteren wird auf diese Weise eine Steigerung der Stabilität und Haltbarkeit erreicht.

Ein weiterer Gegenstand der vorliegenden Erfindung ist eine Elektrolytmischung, umfassend die Ionenanteile a) 24.5 - 25.9 Gew. % Natrium, b) 1.21 - 1.51 Gew. % Kalium, c) 0.15 - 0.61 Gew. % Calcium, d) 0.19 - 0.28 Gew. % Magnesium, e) 27.3 - 29.5 Gew. % Chlorid, f) 0.18 - 0.54 Gew. % Phosphat, g) 27.1 - 39.1 Gew. % Glukonat und h) 9.10 - 12.7 Gew. % Acetat, wobei sich die Angaben in Gew. % jeweils auf das Gesamtgewicht der Elektrolytmischung beziehen.

Erfindungsgemäß werden dabei vorteilhafterweise solche Salze eingesetzt, die in einschlägigen Arzneibüchern empfohlen und monographiert sind, wie u.a. dem europäischen Arzneibuch/European Pharmacopeia und der United States Pharmacopeia.

In einer bevorzugten Ausführungsform werden daher Salze eingesetzt, die aus derjenigen Gruppe ausgewählt sind, die Natriumchlorid, Natriumacetat x 3 H₂O, Natriumhydrogenphosphat x 2 H₂O, D-Gluconsäure Natriumsalz, Kaliumchforid, Kaliumacetat, Calcium-D-gluconat x H₂O und Magnesiumchlorid x 6 H₂O umfasst.

Die erfindungsgemäße Elektrolytmischung lässt sich durch Auflösen in Wasser unter Adaptierung des pH-Werts in die erfindungsgemäße Elektrolytlösung überführen.

Ein weiterer Erfindungsgegenstand ist daher ein Verfahren zur Herstellung der erfindungsgemäßen wässrigen Vollelektrolytlösung, umfassend die Schritte: (i) Lösen der erfindungsgemäßen Elektrolytmischung in einer solchen Menge Wasser, die ausreicht, um die molaren Konzentrationen der jeweiligen Elektrolyte einzustellen; und (ii) Einstellen der Lösung auf einen pH-Wert im Bereich von 5,0 bis 8,0.

Die erfindungsgemäße Elektrolytlösung eignet sich aufgrund ihrer plasmaadaptierten und balancierten Zusammensetzung, Isotonie und unproblematischen lokalen Verträglichkeit sehr gut als Infusionslösung und zwar nicht nur im Rahmen der intravenösen, sondern auch der subkutanen Verabreichung ("Hypodermoklyse"). Gegenstand der vorliegenden Erfindung ist daher die Verwendung der wässrigen Vollelektrolytlösung als intravenöse oder subkutane Infusionslösung. Als solche kann sie, wie gefunden wurde, vorteilhaft zur Behandlung der hypotonen oder isotonen Dehydratation, zur Behandlung von extrazellulären Flüssigkeitsverlusten, Hypovolämie oder Schock und zur Rehydratation des Interstitiums nach kolloidalem Volumenersatz oder als Trägerlösung für kompatible Elektrolyte, Nährstoffe und Medikamente eingesetzt werden.

Ein weiterer Erfindungsgegenstand ist daher ein Arzneimittel, umfassend die wässrige Vollelektrolytlösung und gegebenenfalls eine oder mehrere weitere Komponenten ausgewählt aus der Gruppe bestehend aus Aminosäuren, Kohlenhydraten, Vitaminen, Mineralstoffen, Hydroxyethylstärke, Gelatine, Albumin und zur Infusion bestimmten Medikamenten, vorzugsweise ausgewählt aus der Gruppe bestehend aus Antibiotika, Analgetika, Sedativa, Neuroleptika, Antiemetika, Opiate, Muskelrelaxantien, Katecholamine und andere kreislaufwirksame Medikamente.

Das erfindungsgemäße Arzneimittel ist bevorzugt ein Arzneimittel zur Behandlung der hypotonen oder isotonen Dehydratation, zur Behandlung extrazellulärer Flüssigkeitsverluste, zur Behandlung bei Hypovolämie oder Schock, und zur Rehydratation des Interstitiums nach kolloidalem Volumenersatz.

Ein weiterer Gegenstand der vorliegenden Erfindung ist die Verwendung der wässrigen Vollelektrolytlösung zum Spülen und Reinigen von Instrumenten oder als Trägerlösung für kompatible Elektrolyte, Nährstoffe und Medikamente.

Ein weiterer Gegenstand der vorliegenden Erfindung ist eine wässrigen Vollelektrolytlösung gemäß mindestens einem der Ansprüche 1 bis 9 zur Verwendung als topische Spüllösung beispielsweise zur Spülung und Reinigung bei chirurgischen Eingriffen, zur Spülung und Reinigung von Wunden und Verbrennungen, zur Spülung von Körperhöhlen, zur Augenspülung und zum Spülen und Reinigen bei der Stomaversorgung.

### Beispiele

Die in der folgenden Tabelle 2 angeführten Beispiele sind erfindungsgemäße Elektrolytlösungen.

**Tabelle 2: Herstellungsbeispiele.**

| **Komponenten** | **Beispiel 1:** | **Beispiel 2:** | **Beispiel 3:** | **Beispiel 4:** |
|---|---|---|---|---|
| Natriumchlorid | 5.815 g | 6.195 g | 5.639 g | 5.902 g |
| Natriumacetat x 3 H₂O | 3.266 g | 2.314 g | 2.382 g | 3.539 g |
| Natriumdihydrogenphosphat x 2 H₂O | 0.156 g | 0.156 g | 0.156 g | 0.234 g |
| D-Gluconsäure Natriumsalz | 3.817 g | 4.799 g | 5.454 g | 3.381 g |
| Kaliumchlorid | 0.298 g | - | 0.186 g | 0.186 g |
| Kaliumacetat | - | 0.491 g | 0.245 g | 0.196 g |
| Calcium-D-gluconat x H₂O | 0.561 g | 0.897 g | 0.224 g | 0.561 g |
| Magnesiumchlorid x 6 H₂O | 0.203 g | 0.203 g | 0.305 g | 0.254 g |
| Aqua ad iniectabilia | ad 1000 ml | ad 1000 ml | ad 1000 ml | ad 1000 ml |

Die vorstehend aufgeführten Substanzen werden in den beispielhaft angegebenen Mengen in *aqua ad iniectabilia* unter Rühren vollständig gelöst. Danach werden der pH-Wert mit einem Gemisch aus 10 ml 2 N Salzsäure und 5 ml 2 N Essigsäure auf pH = 6.5 eingestellt, das Volumen mit *aqua ad iniectabila* auf 1000 ml aufgefüllt und die Lösung in Infusionsglasflaschen zu 250 ml abgefüllt, mit Stopfen versehen und verbördelt. Die Flaschen werden in bekannter, dem Stand der Technik entsprechender Weise (z. B. 20 Minuten bei 121 °C) sterilisiert.

## Patentansprüche

1. Wässrige Vollelektrolytlösung, umfassend die Ionenanteile
a)138 - 146 mmol/l Natrium,
b) 4 -5 mmol/l Kalium,
c) 0.5 - 2.0 mmol/l Calcium,
d) 1.0 - 1.5 mmol/l Magnesium,
e) 100 - 108 mmol/l Chlorid,
f) 0.5 - 1.5 mmol/l Phosphat,
g) 18 - 26 mmol/l Glukonat und
h) 20 - 28 mmol/l Acetat.

2. Wässrige Vollelektrolytlösung gemäss Anspruch 1, umfassend 140 - 144 mmol/l Natrium.

3. Wässrige Vollelektrolytlösung gemäss den Ansprüchen 1 oder 2, umfassend 4,3 - 4,7 mmol/l Kalium.

4. Wässrige Vollelektrolytlösung gemäss mindestens einem der vorangehenden Ansprüche, umfassend 1,0 - 1,5 mmol/l Calcium.

5. Wässrige Vollelektrolytlösung gemäss mindestens einem der vorangehenden Ansprüche, umfassend 1,1 - 1,4 mmol/l Magnesium.

6. Wässrige Vollelektrolytlösung gemäss mindestens einem der vorangehenden Ansprüche, umfassend 102 - 106 mmol/l Chlorid.

7. Wässrige Vollelektrolytlösung gemäss mindestens einem der vorangehenden Ansprüche, umfassend 0,8 - 1,2 mmol/l Phosphat.

8. Wässrige Vollelektrolytlösung gemäss mindestens einem der vorangehenden Ansprüche, umfassend g) 20 - 24 mmol/l Glukonat und/oder h) 22 - 26 mmol/l Acetat.

9. Wässrige Vollelektrolytlösung gemäss mindestens einem der vorangehenden Ansprüche, **dadurch gekennzeichnet, dass** sie einen pH-Wert im Bereich von 5,0 bis 8,0 oder 6,0 bis 7,0 aufweist.

10. Elektrolytmischung, umfassend die Ionenanteile
a) 24.5 - 25.9 Gew. % Natrium,
b) 1.21 - 1.51 Gew. % Kalium,
c) 0.15 - 0.61 Gew. % Calcium,
d) 0.19 - 0.28 Gew. % Magnesium,
e) 27.3 - 29.5 Gew. % Chlorid,
f) 0.18 - 0.54 Gew. % Phosphat,
g) 27.1 - 39.1 Gew. % Glukonat und
h) 9.10 - 12.7 Gew. % Acetat, wobei sich die Angaben in Gew. % jeweils auf das Gesamtgewicht der Elektrolytmischung beziehen.

11. Verfahren zur Herstellung der wässrigen Vollelektrolytlösung gemäß mindestens einem der Ansprüche 1 bis 9, umfassend die Schritte:
Lösen der Elektrolytmischung gemäß Anspruch 10 in einer solchen Menge Wasser, die ausreicht, um die molaren Konzentrationen der jeweiligen Elektrolyte einzustellen; und
Einstellen der Lösung auf einen pH-Wert im Bereich von 5,0 bis 8,0.

12. Wässrige Vollelektrolytlösung gemäß mindestens einem der Ansprüche 1 bis 9 zur Verwendung als Infusionslösung, insbesondere als intravenöse oder subkutane Infusionslösung oder zur Verwendung als topische Spüllösung beispielsweise zur Spülung und Reinigung bei chirurgischen Eingriffen, zur Spülung und Reinigung von Wunden und Verbrennungen, zur Spülung von Körperhöhlen, zur Augenspülung und zum Spülen und Reinigen bei der Stomaversorgung.

13. Arzneimittel, umfassend die wässrige Vollelektrolytlösung gemäß mindestens einem der Ansprüche 1 bis 9 und gegebenenfalls eine oder mehrere weitere Komponenten ausgewählt aus der Gruppe bestehend aus Aminosäuren, Kohlenhydraten, Vitaminen, Mineralstoffen, Hydroxyethylstärke, Gelatine, Albumin und zur Infusion bestimmten Medikamenten, wie z. B. Antibiotika, Analgetika, Sedativa, Neuroleptika, Antiemetika, Opiate, Muskelrelaxantien, Katecholamine und andere kreislaufwirksame Medikamente.

14. Arzneimittel gemäß Anspruch 13, zur Verwendung in der Behandlung der hypotonen oder isotonen Dehydratation, zur Behandlung extrazellulärer Flüssigkeitsverluste, zur Behandlung bei Hypovolämie oder Schock, und zur Rehydratation des Interstitiums nach kolloidalem Volumenersatz.

15. Verwendung der wässrigen Vollelektrolytlösung gemäß mindestens einem der Ansprüche 1 bis 9 zum Spülen und Reinigen von Instrumenten oder als Trägerlösung für kompatible Elektrolyte, Nährstoffe und Medikamente.

## Claims

1. A balanced aqueous electrolyte solution, comprising the following proportions of ions:
a) from 138 to 146 mmol/l sodium,
b) from 4 to 5 mmol/l potassium,
c) from 0.5 to 2.0 mmol/l calcium,
d) from 1.0 to 1.5 mmol/l magnesium,
e) from 100 to 108 mmol/l chloride,
f) from 0.5 to 1.5 mmol/I phosphate,
g) from 18 to 26 mmol/l gluconate, and
h) from 20 to 28 mmol/l acetate.

2. The balanced aqueous electrolyte solution according to claim 1, comprising from 140 to 144 mmol/I sodium.

3. The balanced aqueous electrolyte solution according to claims 1 or 2, comprising from 4.3 to 4.7 mmol/l potassium.

4. The balanced aqueous electrolyte solution according to at least one of the preceding claims, comprising from 1.0 to 1.5 mmol/l calcium.

5. The balanced aqueous electrolyte solution according to at least one of the preceding claims, comprising from 1.1 to 1.4 mmol/I magnesium.

6. The balanced aqueous electrolyte solution according to at least one of the preceding claims, comprising from 102 to 106 mmol/I chloride.

7. The balanced aqueous electrolyte solution according to at least one of the preceding claims, comprising from 0.8 to 1.2 mmol/l phosphate.

8. The balanced aqueous electrolyte solution according to at least one of the preceding claims, comprising g) from 20 to 24 mmol/l gluconate, and/or h) from 22 to 26 mmol/l acetate.

9. The balanced aqueous electrolyte solution according to at least one of the preceding claims, **characterized by** having a pH within a range of from 5.0 to 8.0, or from 6.0 to 7.0.

10. An electrolyte mixture, comprising the following proportions of ions:
a) from 24.5 to 25.9% by weight sodium,
b) from 1.21 to 1.51% by weight potassium,
c) from 0.15 to 0.61% by weight calcium,
d) from 0.19 to 0.28% by weight magnesium,
e) from 27.3 to 29.5% by weight chloride,
f) from 0.18 to 0.54% by weight phosphate,
g) from 27.1 to 39.1% by weight gluconate, and
h) from 9.10 to 12.7% by weight acetate, the percentages by weight each being based on the total weight of the electrolyte mixture.

11. A process for preparing the balanced aqueous electrolyte solution according to at least one of claims 1 to 9, comprising the steps of:
dissolving the electrolyte mixture according to claim 10 in an amount of water sufficient to adjust the molar concentrations of the respective electrolytes; and
adjusting the solution to a pH value within a range of from 5.0 to 8.0.

12. The balanced aqueous electrolyte solution according to at least one of claims 1 to 9 for use as an infusion solution, especially as an intravenous or subcutaneous infusion solution, or for use as a topical rinsing solution, for example, for irrigation and cleaning during surgical operations, for the irrigation and cleaning of wounds and burns, for the irrigation of body cavities, for eye rinsing, for the rinsing and cleaning in stoma care.

13. A medicament comprising the balanced aqueous electrolyte solution according to at least one of claims 1 to 9 and optionally one or more further components selected from the group consisting of amino acids, carbohydrates, vitamins, minerals, hydroxyethyl starch, gelatin, albumin and drugs intended for infusion, such as antibiotics, analgetics, sedatives, neuroleptics, antiemetics, opiates, muscle relaxants, catecholamines, and other drugs with cardiovascular action.

14. The medicament according to claim 13 for use in the treatment of hypotonic or isotonic dehydration, for treating extracellular fluid losses, for treatment in hypovolemia or shock, and for rehydrating the interstitial space after colloidal volume replacement.

15. Use of the balanced aqueous electrolyte solution according to at least one of claims 1 to 9 for the irrigation and cleaning of instruments, or as a carrier solution for compatible electrolytes, nutrients and medicaments.

## Revendications

1. Solution électrolytique complète aqueuse, comprenant les concentrations ioniques suivantes :
a) 138-146 mmol/l de sodium,
b) 4-5 mmol/l de potassium,
c) 0,5-2,0 mmol/l de calcium,
d) 1,0-1,5 mmol/l de magnésium,
e) 100-108 mmol/l de chlorure,
f) 0,5-1,5 mmol/l de phosphate,
g) 18-26 mmol/l de gluconate et
h) 20-28 mmol/l d'acétate.

2. Solution électrolytique complète aqueuse selon la revendication 1, comprenant 140-144 mmol/l de sodium.

3. Solution électrolytique complète aqueuse selon la revendication 1 ou 2, comprenant 4,3-4,7 mmol/l de potassium.

4. Solution électrolytique complète aqueuse selon l'une au moins des revendications précédentes, comprenant 1,0-1,5 mmol/l de calcium.

5. Solution électrolytique complète aqueuse selon l'une au moins des revendications précédentes, comprenant 1,1-1,4 mmol/l de magnésium.

6. Solution électrolytique complète aqueuse selon l'une au moins des revendications précédentes, comprenant 102-106 mmol/l de chlorure.

7. Solution électrolytique complète aqueuse selon l'une au moins des revendications précédentes, comprenant 0,8-1,2 mmol/l de phosphate.

8. Solution électrolytique complète aqueuse selon l'une au moins des revendications précédentes, comprenant g) 20-24 mmol/l de gluconate et/ou h) 22-26 mmol/l d'acétate.

9. Solution électrolytique complète aqueuse selon l'une au moins des revendications précédentes, **caractérisée en ce qu'**elle possède un pH compris entre 5,0 et 8,0, ou entre 6,0 et 7,0.

10. Mélange électrolytique, comprenant les concentrations ioniques suivantes :
a) 24,5-25,9 % en poids de sodium,
b) 1,21-1,51 % en poids de potassium,
c) 0,15-0,61 % en poids de calcium,
d) 0,19-0,28 % en poids de magnésium,
e) 27,3-29,5 % en poids de chlorure,
f) 0,18-0,54 % en poids de phosphate,
g) 27,1-39,1 % en poids de gluconate et
h) 9,10-12,7 % en poids d'acétate, les pourcentages en poids étant basés sur le poids total du mélange électrolytique.

11. Procédé pour préparer la solution électrolytique complète aqueuse selon l'une au moins des revendications 1 à 9, comprenant les étapes consistant à :
dissoudre le mélange électrolytique selon la revendication 10 dans une quantité d'eau suffisante pour ajuster les concentrations molaires des électrolytes respectifs ; et
ajuster le pH de la solution à une valeur comprise entre 5,0 et 8,0.

12. Solution électrolytique complète aqueuse selon l'une au moins des revendications 1 à 9, destinée à être utilisée comme solution de perfusion, notamment comme solution de perfusion intraveineuse ou sous-cutanée, ou destinée à être utilisée comme solution d'irrigation topique, par exemple pour l'irrigation et le nettoyage pendant les interventions chirurgicales, pour l'irrigation et le nettoyage des blessures et des brûlures, pour l'irrigation des cavités corporelles, pour le rinçage des yeux, et pour le rinçage et le nettoyage pendant les soins des stomies.

13. Médicament, comprenant la solution électrolytique complète aqueuse selon l'une au moins des revendications 1 à 9 et éventuellement un ou plusieurs autres composants choisis dans le groupe consistant en acides aminés, glucides, vitamines, minéraux, amidon hydroxyéthylé, gélatine, albumine et des médicaments destinés pour la perfusion, tels que les antibiotiques, les analgétiques, les sédatifs, les neuroleptiques, les antiémétiques, les opiacés, les relaxants de muscle, les catécholamines et autres médicaments ayant des effets cardiovasculaires.

14. Médicament selon la revendication 13 destiné à être utilisé dans le traitement de la déshydratation hypotonique ou isotonique, dans le traitement des pertes de liquide extracellulaire, dans le traitement de la hypovolémie ou du choc, et dans la réhydratation de l'interstitium après le remplacement de volume colloïdal.

15. Utilisation de la solution électrolytique complète aqueuse selon l'une au moins des revendications 1 à 9 pour le rinçage et le nettoyage d'instruments ou comme solution conductrice pour électrolytes, nutriments et médicaments compatibles.
